# EUROPEAN PATENT SPECIFICATION

(11) **EP 1 494 661 B1**
(45) Date of publication and mention of the grant of the patent: **14.10.2009**
(21) Application number: 03746454.2
(22) Date of filing: 11.04.2003
(51) Int. Cl.: A61K 31/22, A61K 31/365, A61K 31/40, A61K 31/404, A61P 19/02, A61P 37/00

(54) **PTX3 GENE EXPRESSION SUPPRESSING METHOD**
VERFAHREN ZUR UNTERDRÜCKUNG DER PTX3 GENEXPRESSION
PROCEDE DE SUPPRESSION DE L'EXPRESSION DE GENE PTX3

(30) Priority: 15.04.2002 US 372114 P; 17.07.2002 US 196428
(43) Date of publication of application: 12.01.2005
(73) Proprietor: Kowa Co., Ltd., Nagoya-shi, Aichi 460-8625 (JP); Nissan Chemical Industries, Ltd., Chiyoda-ku, Tokyo 101-0054 (JP)
(72) Inventor: MORIKAWA, Shigeru, TOSHIMA-KU, Tokyo 171-0033 (JP); IZUMI, Akashi, MACHIDA-SHI, Tokyo 195-0061 (JP); HAMAKUBO, Takao, KOMAE-SHI, Tokyo 201-0005 (JP); KODAMA, Tatsuhiko, SETAGAYA-KU, Tokyo 154-0002 (JP)
(74) Representative: Hartz, Nikolai
(86) International application number: PCT/JP2003/004603
(87) International publication number: WO 2003/086380

(56) References cited:
- EP-A- 1 275 388
- WO-A-00/53566
- WO-A-02/24194
- WO-A-99/26657
- WO-A1-03/061702
- PITAVASTATIN: ITAVASTATIN, NISVASTATIN, NK 104, NKS 104, P 872441 DRUGS IN R&D vol. 3, no. 1, 2002, pages 58 - 60

### SEQUENCE LISTING

<110> KOWA CO., LTD.
<120> PTX3 GENE EXPRESSION SUPPRESSING METHOD
<130> KW0101
<140>
   <141>
<150> US 60/372,114
   <151> 2002-04-15
<150> US 10/196,428
   <151> 2002-07-17
<160> 4
<210> 1
   <211> 21
   <212> DNA
   <213> Artificial Sequence
<220>
   <223> Description of Artificial Sequence: Designedprimer PTX3 (forward)
<400> 1
   agtgtttgtg gtgggtggag a 21
<210> 2
   <211> 21
   <212> DNA
   <213> Artificial Sequence
<220>
   <223> Description of Artificial Sequence: Designedprimer PTX3 (Reverse)
<400> 2
   ttatgaaaca tactgagctc c 21
<210> 3
   <211> 23
   <212> DNA
   <213> Artificial Sequence
<220>
   <223> Description of Artificial Sequence: Designed primer GAPDH (forward)
<400> 3
   acaactttgg tatcgtggaa gga 23
<210> 4
   <211> 22
   <212> DNA
   <213> Artificial Sequence
<220>
   <223> Description of Artificial Sequence: Designed primer GAPDH (Reverse)
<400> 4
   ccgttcagct cagggatgac ct 22

## Description

### Technical Field

This invention relates to the use of a pentraxin 3 (PTX3) gene expression suppressor, pitavastatin for the treatment of autoimmune diseases, especially rheumatoid arthritis.

### Background Art

WO 02/24194 discloses specific HMG-CoA reductase inhibitors for use as immuno-modulators, immuno-suppressors and anti-inflammatory agents.

PTX3 gene was found as a novel gene the expression of which is induced by interleukin-1 (IL-1) from normal human umbilical vein endothelial cells (HUVEC) [Breviario et al.: J. Biol. Chem., 267(31), 22190-7 (1992)]. Further, a gene (TSG-14 gene) the expression of which is induced by tumor necrosis factor α (TNF-α) from human fibroblasts was also found [Lee et al.: J. Immunol., 150(5), 1804-12 (1993)], and from a structural analysis, this gene has been found to be the same as PTX3 gene. PTX3 protein, in view of its molecular structure, belongs to the so-called pentraxin family such as C-reactive protein (CRP) and serum amyloid P component (SAP), but its physiological functions are not known much. For reasons such that PTX3 protein is not induced by IL-6 and is different from the species of cells to be produced, PTX3 protein was suggested to have functions different from CRP or SAP [J. Biol. Chem., 267(31), 22190-7 (1992); Domyaku Koka (Arteriosclerosis), 24(7-8), 375-80 (1996)].

As relevancy to the inflammatory reaction such as a formation of an arteriosclerotic layer or an ischemic heart disease, it has been found that the blood level of PTX3 is high in acute myocardial infarction patients [Circulation, 102, 636-41 (2000)] and that expression of a tissue factor, an important factor for the formation of thrombus, is increased byPTX3 [Arterioscler. Thromb. Vasc. Biol., 22, 782-7 (2002)].

Recently, it has also been revealed that PTX3 gene is constantly expressed in synovial cells of a rheumatoid arthritis patient and that this expression is suppressed by inteferon-γ (IFN-γ) or transforming growth factor-β (TGF-β) [Clin. Exp. Immunol., 119(1), 196-202 (2000)]. Moreover, PTX3 also takes part in a disorder via a complement pathway in an autoimmune disease, especially rheumatoid arthritis, because it binds to C1q, one of complement components, to activate the complement pathway [J. Biol. Chem., 272(52), 32817-23 (1997)].

Suppression of PTX3 gene expression, therefore, suppresses worsening of an autoimmune disease, especially rheumatoid arthritis and further, results in its treatment. Except for IFN-γ and TGF-β, however, absolutely no substance has been known to date to suppress expression of PTX3 gene.

An object of the present invention is, therefore, to provide a PTX3 gene expression suppressor, which suppresses expression of PTX3 gene and is effective for the treatment of an autoimmune disease, especially rheumatoid arthritis.

### Disclosure of the Invention

Using a cultured human cell system, the present inventors have hence looked for substances which affect expression of PTX3 gene. As a result, it has been quite unexpectedly found that pitavastatin calcium has activities to suppress expression of PTX3 gene, leading to the completion of the present invention.

The present invention also provides use as defined by claim 1.

### Brief Description of the Drawings

FIG. 1 is a diagram showing expression levels of PTX3 gene; and
FIG. 2 is a diagram electrophoretically illustrating suppression of gene expression.

### Best Modes for Carrying Out the Invention

HMG-CoA reductase suppressors are useful as hyperlipidemia therapeutics. However, absolutely nothing is known as to whether or not they affect expression of PTX3 gene.

The salts of pitavastatin are physiologically acceptable salts. Examples can include alkali metal salts such as the sodium salts and potassium salts, alkaline earth metal salts such as the calcium salts and magnesium salts, organic amine salts such as the phenethylamine salts, and the ammonium salts, with the sodium salts and calcium salts being more preferred.

Compounds are disclosed, for example, in US-A-4,739,073 and EP-A-114,027; EP-A-367,895; US-A-5,001,255, US-A-4,613,610, US-A-4,851,427, US-A-4,755,606, US-A-4,808,607, US-A-4,751,235, US-A,4,939,159, US-A-4,822,799, US-A-4,804,679, US-A-4,876,280, US-A-4,829,081, US-A-4,927,851, US-A-4,588,715; F.G.kathawala, Medical Research Reviews, 11, 121-146 (1991), EP-A-304,063; EP-A-330,057; US-A-5,026,708, US-A-4,868,185; EP-A-324,347; EP-A-300, 278; US-A-5,013,749, US-A-5,872,130, US-A-5,856,336, US-A-4,231,938, US-A-4,444,784, US-A-4,346,227, US-A-5,354,772, US-A-5,273,995, US-A-5,177,080, US-A-3,983,140, JP-B-2,648,897, US-A-5,260,440, Bioorganic & Medicinal Chemistry, 5, 437 (1977), JP-B-2,569,746, EP-B-304,063, and UA-5,856,336.

The active ingredient in the medicament according to the present invention for the suppression of expression of PTX3 gene is pitavastatin (US-A-5,856,336, JP-B-2,569,746:
(3R,5S,6E)-7-[2-cyclopropyl-4-(4-fluorophenyl)-3-quinolyl ]-3,5-dihydroxy-6-heptenoic acid, and their salts. In particular, pitavastatin and its salts are used, all of which significantly suppress expression of mRNA for PTX3 in human cells and therefore, are useful in the PTX3 gene expression suppressor, especially for the treatment of autoimmune diseases such as rheumatoid arthritis. Further, they also permit inter alia development of experiment systems, in which PTX3 takes part, and screening of novel medicines.

Illustrative administration routes for the compound (1) or its lactone or the salt of the compound or lactone can include oral administrations by tablets, capsules, a granule, apowder, a syrup and the like; and parenteral administrations by an intravenous injection, an intramuscular injection, suppositories, an inhalant, a transdermal preparation, an eye drop, a nasal drop and the like.

To formulate medicinal preparations in such various forms as described above, the active ingredient can be used either singly or in combination with one or more of pharmaceutically acceptable excipients, binders, extenders, disintegrants, surfactants, lubricants, dispersants, buffering agents, preservatives, corrigents, perfumes, coating materials, carriers, diluents and the like, as needed.

Of these administration routes, oral administrations are preferred. Upon formulation of a medicinal preparation for oral administration, it is preferred to adjust the pH in view of the stability of the active ingredient (JP-A-2-0006406, JP-B-2,774,037, WO-A-97/23200, etc.).

The dosage of the active ingredient varies inter alia depending on the weight, age, sex and conditions of each patient. In the case of an adult, however, it is generally preferred to orally or parenterally administer the active ingredient at a daily dosage of from 0.01 to 1,000 mg, specifically from 0. 1 to 100 mg in terms of the compound represented by formula (1) at once or in several portions.

### Examples

The present invention will hereinafter be described in detail based on Examples.

### Example 1 and Reference Example 1

Two days after inoculation of normal human umbilical vein endothelial cells (HUVEC) or human coronary artery smooth muscle cells (HCASMC) at 3 x 10⁵ cells/10 cm dish, pitavastatin calcium or atorvastatin calcium (Reference Example) was added to 1.1 µmol/L and 6.6 µmol/L, respectively. Dimethyl sulfoxide, a solvent for both of the active ingredients, was added to a control (final concentration: 0.0066v/v%) Eight hours after the addition, total RNA was extracted with "ISOGEN" (trade mark, product of NIPPON GENE CO., LTD.). The following procedures was conducted in accordance with the procedures manual of Affymetrix. Inc. Described specifically, mRNA was isolated from the above-obtained total RNA, and based on the mRNA, cDNA was synthesized. Further, biotin-labeled cRNA was synthesized by in vitro transcription. Subsequent to purification, the biotin-labeled cRNA was subjected to fragmentation by heat treatment to prepare fragmented cRNA for use in a gene expression analysis.

Gene expression analysis method: The fragmented cRNA was poured into "Hugene Human FL Array" (trade name, product of Affymetrix, Inc.), and hybridization was conducted at 45°C for 16 hours. Subsequent to washing, staining with phycoerythrin-labeled streptavidin and biotinylated antistreptavidin antibody was applied, and gene expression information was inputted by "GeneChip^{™} Scanner" (trade name, manufactured by Hewlett Packard Company). The information was analyzed by "GENECHIP SOFTWARE" (trade name, product of Affymetrix, Inc.) to compare expression levels.

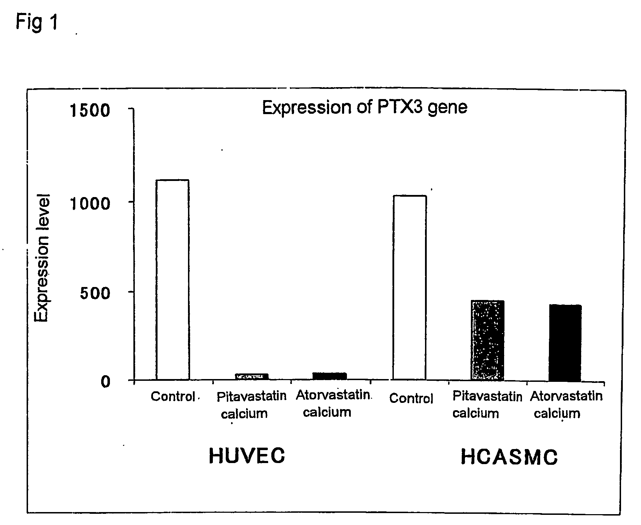

The results of the measurement are shown in FIG. 1.

The expression of PTX3 gene in HUVEC upon elapsed time of 8 hours after the addition of the active ingredient was significantly suppressed to 32.7 and 39.2 in the pitavastatin calcium and atorvastatin calcium addition groups, respectively, as opposed to 1113.0 in the control. The expression of PTX3 gene in HCASMC upon elapsed time of 8 hours after the addition of the active ingredient was also significantly suppressed to 452.5 and 432.1 in the pitavastatin calcium and atorvastatin calcium addition groups, respectively, as opposed to 1028.3 in the control.

### Example 2 and Reference Example 2

Two days after inoculation of HUVEC at 3 x 10⁵ cells/10 cmdish, pitavastatin calciumor atorvastatin calcium (Reference Example) was added to 1.1 µmol/L and 6.6 µmol/L, respectively. To ascertain possible concentration dependency of PTX3 gene expression suppressing effect of pitavastatin, pitavastatin calcium was also added to 1 µmol/L and 10 µmol/L upon elapsed time of 2 days after inoculation of HUCEC or HCASMC at 3 x 10⁵ cells/10 cm dish. To controls under the respective conditions, dimethyl sulfoxide, a solvent for both of the active ingredients, was added (final concentration: 0.0066 v/v%). Eight or 24 hours after the addition, total RNA was extracted with "ISOGEN" (trade mark, product of NIPPON GENE CO., LTD.). The total RNA was subjected to RT-PCR in a manner known per se in the art, and amplified DNA fragments were subjected to agarose gel electrophoresis to compare expression levels.

### Reaction conditions and the like for PT-PCR:

PT reaction: Conducted using "RNA PCR Core Kit" (trade name, product of Roche Molecular Systems, Inc.).
PCR: Using "Expanded^{™} High Fidelity PCR System" (trade name, manufactured of Boehringer Mannheim AG), thermal cycling was conducted through 25 cycles according to the following schemes: 95°C for 1 minute - 57°C for 1 minute - 72°C for 1 minute.
Incidentally, as PCR primers, the followings were used in sets: SEQ ID No:1 (Forward) and SEQ ID No: 2 (Reverse) in the case of PTX3; base SEQ ID No:3 (Forward) and SEQ ID No: 4 (Reverse) in the case of GAPDH.

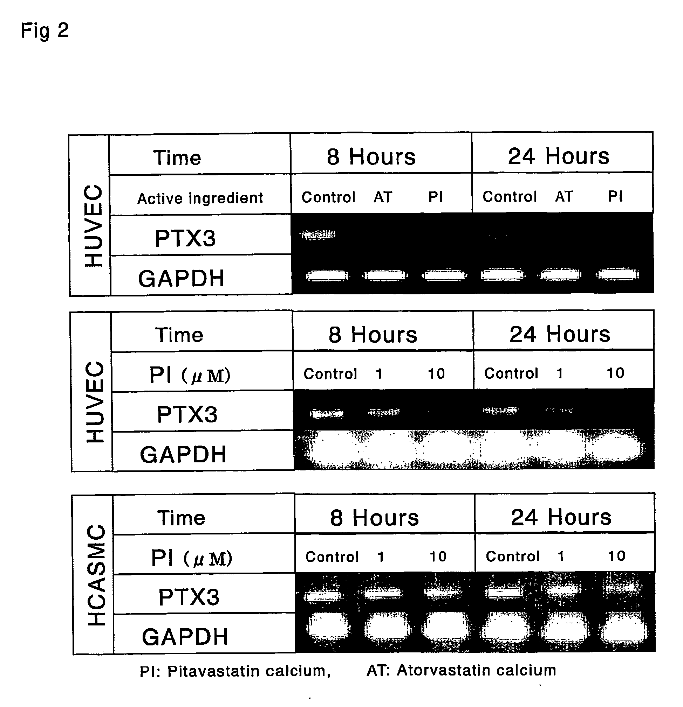

The results are shown in FIG. 2.

The expression of PTX3 gene in HUVEC was suppressed by the addition of pitavastatin calcium or atorvastatin calcium both 8 hours later and 24 hours later compared with the control. Further, the expressions of PTX3 gene in HUVEC and HCASMC were concentration-dependently suppressed by the addition of pitavastatin calcium both 8 hours later and 24 hours later.

### Industrial Applicability

The present invention can provide a PTX3 gene expression suppressor namely pitavastatin useful for the treatment of autoimmune diseases, especially rheumatoid arthritis.

## Claims

1. Use of pitavastatin or a salt thereof in the preparation of a medicament for administration to a patient so as to suppress pentraxin 3 (PTX3) gene expression in the treatment of autoimmune disease.

2. The use according to claim 1, wherein the autoimmune disease is rheumatoid arthritis.

3. The use according to claim 1, wherein the suppression of pentraxin 3 gene expression occurs in a dose dependent manner.

## Patentansprüche

1. Verwendung von Pitavastatin oder einem Salz davon zur Herstellung eines Medikaments zur Verabreichung an einen Patienten, um die Expression eines Pentraxin 3 (PTX3) Gens bei der Behandlung von Autoimmunkrankheiten zu Unterdrücken.

2. Die Verwendung nach Anspruch 1, wobei die Autoimmunkrankheit rheumatoide Arthritis ist.

3. Die Verwendung nach Anspruch 1, wobei die Unterdrückung der Expression eines Pentraxin 3 Gens in einer dosisabhängigen Weise erfolgt.

## Revendications

1. Utilisation de la pitavastatine ou d'un sel de celle-ci dans la préparation d'un médicament pour administration à un patient de manière à supprimer l'expression du gène pentraxin 3 (PTX3) dans le traitement d'une maladie autoimmune.

2. Utilisation selon la revendication 1, dans laquelle la maladie autoimmune est l'arthrite rhumatoïde.

3. Utilisation selon la revendication 1, dans laquelle la suppression de l'expression du gène pentraxin 3 a lieu d'une manière dose-dépendante.
